# EUROPÄISCHE PATENTSCHRIFT

(11) **EP 2 681 539 B1**
(45) Veröffentlichungstag und Bekanntmachung des Hinweises auf die Patenterteilung: **19.10.2016**
(21) Anmeldenummer: 12716201.4
(22) Anmeldetag: 28.02.2012
(51) Int. Cl.: G01N 27/12

(54) **GASSENSOR, INSBESONDERE FÜR AUTOMOBILE ANWENDUNGEN**
GAS SENSOR, IN PARTICULAR FOR AUTOMOBILE APPLICATIONS
CAPTEUR DE GAZ, EN PARTICULIER POUR APPLICATIONS AUTOMOBILES

(30) Priorität: 01.03.2011 DE 102011012682
(43) Veröffentlichungstag der Anmeldung: 08.01.2014
(73) Patentinhaber: Hella KGaA Hueck & Co, 59552 Lippstadt (DE)
(72) Erfinder: NIEMANN, Thomas, 27753 Dalmenhorst (DE); EGGERS, Torsten, 28215 Bremen (DE)
(74) Vertreter: Siekmann, Gunnar
(86) Internationale Anmeldenummer: PCT/DE2012/000186
(87) Internationale Veröffentlichungsnummer: WO 2012/116681

(56) Entgegenhaltungen:
- WO-A1-2009/068405
- JP-A- 61 195 339
- JP-A- 2008 014 662
- US-A- 5 659 127
- US-B1- 6 565 812

## Beschreibung

Die Erfindung betrifft einen Gassensor, insbesondere einen Gassensor für automobile Anwendungen, mit einem Gehäuse, das eine Messkammer aufweist, mit den Merkmalen des Oberbegriffs des Patentanspruchs 1. Weiterhin betrifft die Erfindung ein Verfahren zur Herstellung eines Gassensors mit den Merkmalen des Oberbegriffs des Patentanspruchs 10.

Ein gattungsgemäßer Gassensor und ein gattungsgemäßes Verfahren sind aus der US 5,659,127 A bekannt. Aus dieser Druckschrift ist eine Substratstruktur für einen Gassensor bekannt. Dieser weist ein Gehäuse mit einer Messkammer auf, wobei in der Messkammer ein Sensorelement vorgesehen ist, das an seinen Anschlussdrähten hängend in einem Unterteil angeordnet ist. Weiterhin ist ein Oberteil vorgesehen, das auf das Unterteil aufgeschoben wird. Das Unterteil weist an seiner Oberkante dreieckige Aussparungen auf, in denen die Anschlussdrähte befestigt werden.

Die JP 61195339 A zeigt einen Gassensor mit einem Sensorelement, das an seinen Anschlussdrähten hängend in einem Unterteil angeordnet ist. Auf einem sehr flachen Unterteil sind stangenförmigen Elementen angeordnet. An diesen ist mit Anschlussdrähten der Hauptkörper des Sensorelements aufgehängt. Eine Kappe wird dann auf das Unterteil aufgesetzt.

Aus der WO 2009/068405 A1 ist ein Gassensor mit einem niedrigen Energieverbrauch bekannt. Ein Metalloxid-Gassensor ist an Bonddrähten hängend ausgeführt. Die Bonddrähte stellen eine thermische Isolierung gegenüber dem Gehäuse des Sensors dar und der Sensor bekommt durch die große Dicke und damit thermische Masse des Sensorchips insgesamt eine thermische Zeitkonstante für das Aufheizen und Abkühlen im Bereich einiger Sekunden. Aus der JP 2008/014662 A sind_ein Gasfilter und ein Gassensor bekannt, bei dem ein Element zwischen Stäben hängend in einer Kammer angeordnet ist. In der US 656812 B1 ist ein Gassensor und ein Herstellungsverfahren hierfür beschrieben. Ein Sensorelement wird an Elektroden aufgehängt bzw. wird von diesen gehalten.

Die DE 101 33 466 B4 gibt einen Gassensor an, welcher in einen Schichtenverbund integriert ist. In der DE 10 2008 034 03 ist ein Trägerelement für einen multifunktionellen Sensor beschrieben.

Gassensoren der vorbezeichneten Gattung werden unter anderem auch im Bereich der Fahrzeugbelüftung verwendet, um die Insassen vor Beeinträchtigungen durch Verunreinigungen der zugeführten Frischluft zu schützen.

Bekannte Gassensoren, insbesondere ihre die Messkammer mit dem eigentlichen Sensorelement umschließenden Gehäuse, weisen durch eine Beheizung des Sensorelements auf über 300° Celsius hochtemperierte Sensorbereiche auf.

Es gilt, die verschiedenen Bauelemente des Gassensors, insbesondere die Gehäuseteile und das Sensorelement, thermisch zu entkoppeln und gleichzeitig den komplexen Aufbau und Montage der bekannten Gassensoren zu vereinfachen.

Diese Aufgabe ist erfindungsgemäß durch die Merkmale des Patentanspruchs 1 gelöst. Verfahrensmäßig wird die Aufgabe mit den Merkmalen des Patentanspruchs 10 gelöst. Weiterbildungen und vorteilhafte Ausgestaltungen der erfindungsgemäßen Lösung ergeben sich aus den Unteransprüchen 2 bis 9.

Der Gassensor, insbesondere für automobile Anwendungen, mit einem Gehäuse, das eine Messkammer aufweist, wobei der Messkammer wenigstens ein Anschlussdrähte aufweisendes Sensorelement zugeordnet ist, wobei das Gehäuse ein Unterteil und ein das Unterteil deckelndes Oberteil aufweist und jedes Sensorelement, an seinen Anschlussdrähten hängend, in dem Unterteil angeordnet ist, zeichnet sich erfindungsgemäß dadurch aus, dass das Unterteil in wenigstens einer seiner Seitenwände eine Montageöffnung aufweist, die derart ausgebildet ist, dass ein das Sensorelement haltender Werkzeugarm durch die Montageöffnung von außen in das Unterteil einführbar ist, und dass das Oberteil über das Unterteil fügbar bzw. im montierten Zustand derart aufgesetzt oder angeordnet ist, dass die Seitenwand des Oberteils die Montageöffnung in der Seitenwand des Unterteils verschließt.

Es ist ein besonderer Vorteil der erfindungsgemäßen Lösung, dass die Bauteile des Gassensors auf ein Unterteil, ein das Unterteil deckelndes Oberteil sowie auf mindestens ein Sensorelement beschränkt sind.

Das Unterteil umschließt die eigentliche Messkammer, die nach Anbringen des deckelnden Oberteils als geschlossener Raum vorliegt. Dadurch wird verhindert, dass die Heizung auf dem das Sensorelement aufweisenden Chip weitere Sensoren und Elektronikteile in der Umgebung nachteilig beeinflusst.

Dies wird insbesondere auch dadurch gewährleistet, dass jedes Sensorelement und damit jeder Chip, an den Anschlussdrähten hängend in dem Unterteil angeordnet ist. Die Anschlussdrähte sind mit Vorteil Platinmanteldrähte an denen der Chip frei in dem geschlossenen Gehäuse hängt, womit sich die Aufheizung auf den Chip, beziehungsweise das Sensorelement, beschränkt.

Das Unterteil weist einen Boden und davon aufgehende Seitenwände auf, deren freie Enden dem oberen Öffnungsrand des Unterteils des Gehäuses entsprechen. In der vom oberen Öffnungsrand des Unterteils beschriebenen Ebene befindet sich das an seinen Anschlussdrähten hängende Sensorelement.

An wenigstens zwei einander gegenüberliegenden Seitenwänden des Unterteils des Gehäuses ist mindestens eine Anschlussfläche angeordnet, die jeweils einen aus dem Gehäuse herausgeführten Anschlussbereich aufweist.

Die freien Enden der Anschlussdrähte des Sensorelements sind zum Öffnungsrand des Unterteils des Gehäuses geführt und dort verankert, beispielsweise mit den dort vorhandenen Anschlussflächen verbunden, die an wenigstens zwei einander gegenüberliegenden Seitenwänden des Unterteils angeordnet sind. Jede Anschlussfläche weist jeweils einen aus dem Gehäuse herausgeführten Anschlussbereich auf, über den die an ihren Anschlussdrähten hängenden Sensorelemente mit einer außerhalb befindlichen Fahrzeugelektronik kommunizieren können. Die Anschlussdrähte und die Anschlussflächen sind miteinander verbunden, insbesondere durch eine Schweißverbindung, insbesondere durch Spaltschweißen. Man spricht hier auch von Bonden oder Drahtbonden.

Erfindungsgemäß ist die Montageöffnung in einer der Seitenwände des Unterteils derart ausgebildet ist, dass ein das Sensorelement haltender Werkzeugarm durch die Montageöffnung von außen in das Unterteil bis in die Messkammer einführbar und in eine Montageposition im Bereich des Öffnungsrandes des Unterteils derart bringbar ist, dass die Anschlussdrähte des Sensorelements mit den zugeordneten Lötanschlussflächen in bondbarem Kontakt stehen.

Dadurch ist die Herstellung des erfindungsgemäßen Gassensors verhältnismäßig einfach und unkompliziert, woraus sich auch Kostenvorteile ergeben. Auch das Oberteil weist ein Deckelteil und Seitenwandteile auf, aus denen ein Außengehäuse mit unterem Öffnungsrand gebildet ist. Aus dem Unterteil ist ein Innengehäuse mit oberem Öffnungsrand ausgebildet. Erfindungsgemäß ist das Oberteil über das Unterteil fügbar, derart, dass die Seitenwand des Oberteils die Montageöffnung in der Seitenwand des Unterteils verschließt. Damit erübrigen sich Maßnahmen zum Verschließen der Montageöffnungen des Gassensors, sobald sein Sensorelement in dem ein Innengehäuse bildenden Unterteil befestigt ist.

Die Abmessungen des Unterteils und des darüber positionierten Oberteils des Gehäuses sind so gewählt, dass zwischen dem das Innengehäuse darstellenden Unterteil und dem das Außengehäuse darstellenden übergestülpten Oberteil ein Luftspalt verbleibt, der eine vorteilhafte Isolierung bewirkt.

Weiterhin ist bei dem erfindungsgemäßen Gassensor vorgesehen, dass das Deckelteil des Oberteils eine Öffnung aufweist, in der ein gasdurchlässiges jedoch flüssigkeitsundurchlässiges Verschlusselement angeordnet ist. Die Messkammer ist dadurch gegen Außeneinflüsse abgeschirmt. Mit Vorteil ist das Verschlusselement eine wasserundurchlässige aber dampfdiffusionsoffene Membran, z.B. aus expandiertem PTFE (ePTFE). Andere Verschlusselemente, die gegen flüssiges Wasser abschirmend wirken, sind ebenfalls einsetzbar.

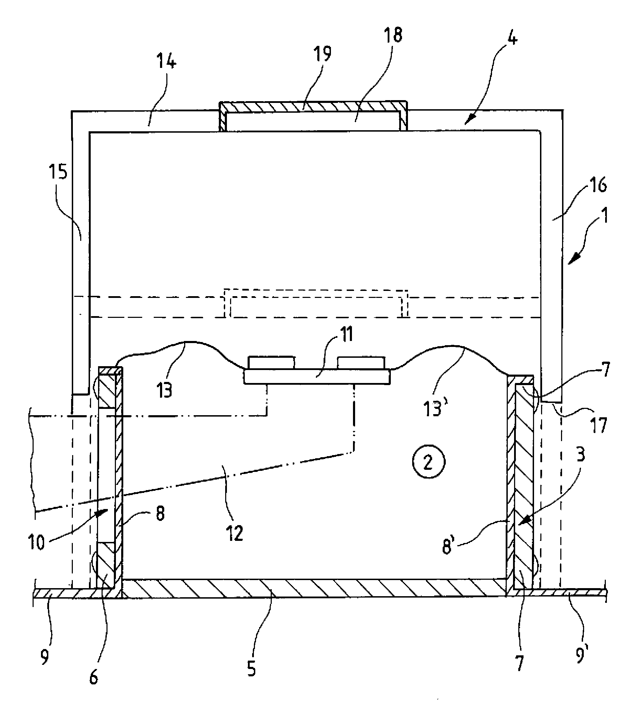

Ein Ausführungsbeispiel der Erfindung, aus dem sich weitere erfinderische Merkmale ergeben, ist in der Zeichnung dargestellt.

Die Zeichnung zeigt eine Seitenansicht eines erfindungsgemäßen Gassensors im Schnitt.

Der Gassensor hat ein Gehäuse 1, das eine Messkammer 2 umschließt und nach außen abschirmt. Das Gehäuse 1 weist ein Unterteil 3, sowie ein das Unterteil deckelnde Oberteil 4 auf. Das Unterteil 3 des Gehäuses besteht aus einem Boden 5 und davon aufgehenden Seitenwänden 6 und 7. Die freien Enden der Seitenwände bilden den oberen Öffnungsrand 7 des Unterteils 3 des Gehäuses 1. An wenigstens zwei einander gegenüberliegenden Seitenwänden 6, 7 des Unterteils 3 des Gehäuses 1 ist mindestens eine Anschlussfläche 8 beziehungsweise 8' angeordnet, die jeweils einen aus dem Gehäuse 1 herausgeführten Anschlussbereich 9, 9' aufweisen.

Das Unterteil 3 weist in seiner Seitenwand 6 eine Montageöffnung 10 auf, die derart ausgebildet ist, dass ein einen Sensor 11 haltender Werkzeugarm 12 in das Unterteil 3, und somit in die Messkammer 2, reichen kann. Damit ist das Sensorelement in eine Montageposition im Bereich, beziehungsweise in der Ebene des Öffnungsrandes 7 des Unterteils bringbar, derart, dass die Anschlussdrähte 13, 13' des Sensorelementes 11 mit den zugeordneten Anschlussflächen 8, 8' in bondbarem Kontakt stehen.

Nach dem Bonden der Anschlussdrähte 13, 13' an die Anschlussflächen der Seitenwände 6, 7 des Unterteils 3 kann der Werkzeugarm 12 durch die Montageöffnung nach außen zurückgezogen werden, wobei das nunmehr an seinen Anschlussdrähten hängende Sensorelement im Unterteil des Gehäuses zurückbleibt.

Anschließend wird das Oberteil 4 des Gehäuses 1 abgesenkt, bis es sich in der durch gestrichelte Linien dargestellten Position befindet. Dabei ist dann die Montageöffnung 10 in der Seitenwand 6 des Unterteils 3 des Gehäuses 1 verschlossen.

Das Oberteil 4 besteht aus einem Deckelteil und Seitenwandteilen 15, 16. Deckelteil und Seitenwandteile bilden ein Außengehäuse mit unterem Öffnungsrand 17.

Das Deckelteil 14 des Oberteils 4 weist eine Öffnung 18 auf, in der ein gasdurchlässiges jedoch flüssigkeitsundurchlässiges Verschlusselement angeordnet ist. Das Verschlusselement kann zum Beispiel eine flüssigkeitsundurchlässige, jedoch dampfdiffusionsoffene Membran 19 sein.

## Patentansprüche

1. Gassensor, insbesondere für automobile Anwendungen, mit einem Gehäuse, das eine Messkammer aufweist, wobei in der Messkammer wenigstens ein Anschlussdrähte aufweisendes Sensorelement enthalten ist, wobei das Gehäuse ein Unterteil (3) und ein das Unterteil (3) deckelndes Oberteil (4) aufweist, und jedes Sensorelement, an seinen Anschlussdrähten (13, 13') hängend, in dem Unterteil (3) angeordnet ist,
**dadurch gekennzeichnet,**
**dass** das Unterteil (3) in wenigstens einer seiner Seitenwände (6) eine Montageöffnung aufweist, die derart ausgebildet ist, dass ein das Sensorelement haltender Werkzeugarm durch die Montageöffnung von außen in das Unterteil (3) einführbar ist und dass das Oberteil (4) derart auf dem Unterteil (3) angeordnet ist, dass die Seitenwand (15, 16) des Oberteils (4) die Montageöffnung (10) in der Seitenwand (6) des Unterteils (3) verschließt.

2. Gassensor nach Anspruch 1, **dadurch gekennzeichnet, dass** das Unterteil (3) einen Boden (5) und davon ausgehende Seitenwände (6) aufweist, deren freie Enden dem oberen Öffnungsrand (7) des Unterteils (3) des Gehäuses (1) entsprechen.

3. Gassensor nach Anspruch 2, **dadurch gekennzeichnet, dass** an wenigstens zwei einander gegenüberliegenden Seitenwänden (6) des Unterteils (3) des Gehäuses (1) mindestens eine Anschlussfläche (8, 8') angeordnet ist, die jeweils einen aus dem Gehäuse (1) herausgeführten Anschlussbereich (9, 9') aufweisen.

4. Gassensor nach einem der Ansprüche 1 bis 3, **dadurch gekennzeichnet, dass** die Montageöffnung (10) derart ausgebildet ist, dass ein das Sensorelement (11) haltender Werkzeugarm (12) in eine Montageposition im Bereich des Öffnungsrandes (7) des Unterteils (3) derart bringbar ist, dass die Anschlussdrähte (13, 13') des Sensorelements (11) mit den zugeordneten Lötanschlussflächen (8, 8') in bondbarem Kontakt stehen.

5. Gassensor nach einem der vorhergehenden Ansprüche, **dadurch gekennzeichnet, dass** das Oberteil (4) ein Deckelteil (14) und Seitenwandteile (15, 16) aufweist, aus denen ein Außengehäuse mit unterem Öffnungsrand (17) gebildet ist.

6. Gassensor nach einem der Ansprüche 1 bis 4, **dadurch gekennzeichnet, dass** das Unterteil (3) als ein Innengehäuse mit oberem Öffnungsrand (7) ausgebildet ist.

7. Gassensor nach Anspruch 6, **dadurch gekennzeichnet, dass** zwischen Unterteil (3) und darüber gefügtem Oberteil (4) ein Luftspalt ausgebildet ist.

8. Gassensor nach einem der vorhergehenden Ansprüche, **dadurch gekennzeichnet, dass** das Deckelteil (14) des Oberteils (4) eine Öffnung (18) aufweist, in der ein gasdurchlässiges jedoch flüssigkeitsundurchlässiges Verschlusselement angeordnet ist.

9. Gassensor nach Anspruch 8, **dadurch gekennzeichnet, dass** das Verschlusselement eine Membran (19) ist.

10. Verfahren zur Herstellung eines Gassensor, insbesondere für automobile Anwendungen, mit einem Gehäuse, das eine Messkammer aufweist, wobei in der Messkammer wenigstens ein Anschlussdrähte aufweisendes Sensorelement enthalten ist, wobei das Gehäuse ein Unterteil (3) und ein das Unterteil (3) deckelndes Oberteil (4) aufweist, und jedes Sensorelement, an seinen Anschlussdrähten (13, 13') hängend, in dem Unterteil (3) angeordnet ist,
**dadurch gekennzeichnet,**
**dass** in dem Unterteil (3) in wenigstens einer seiner Seitenwände (6) eine Montageöffnung (10) vorgesehen ist, und ein das Sensorelement (11) haltender Werkzeugarm durch die Montageöffnung von außen in das Unterteil (3) eingeführt und in eine Montageposition im Bereich des Öffnungrandes (7) des Unterteils gebracht wird, so dass die Anschlussdrähte (13, 13') des Sensorelementes (11) mit den zugeordneten Lötanschlussflächen (8, 8') im bondbaren Kontakt stehen,
**dass** die Anschlussdrähte (13, 13') Anschlussflächen der Seitenwände (6, 7) des Unterteils (3) gebondet werden,
der Werkzeugarm (12) durch die Montageöffnung (10) nach außen zurückgezogen wird und
**dass** das Oberteil (4) auf dem Unterteil (3) des Gehäuses angeordnet wird, so dass die Seitenwand (15, 16) des Oberteils (4) die Montageöffnung (10) in der Seitenwand (6) des Unterteils (3) verschließt.

## Claims

1. A gas sensor, in particular for automobile applications, comprising a housing with a measuring chamber, wherein at least one sensor element having connecting wires is contained in the measuring chamber, wherein the housing has a lower part (3), and an upper part (4) which serves as a cover for the lower part (3), and each sensor element is arranged so as to be suspended by its connecting wires (13, 13') in the lower part (3),
**characterised in**
**that** at least one of the side walls (6) of the lower part (3) has an assembly opening which is designed in such manner that a tool arm holding the sensor element, can be advanced into the lower part (3) from the outside through the assembly opening, and that the upper part (4) is arranged on the lower part (3) in such manner that the side wall (15, 16) of the upper part (4) closes the assembly opening (10) in the side wall (6) of the lower part (3).

2. The gas sensor according to claim 1, **characterised in that** the lower part (3) has a bottom (5) and side walls (6) extending therefrom, the free ends of which side walls correspond to the upper opening perimeter (7) of the lower part (3) of the housing (1).

3. The gas sensor according to claim 2, **characterised in that** at least one connection surface (8, 8') is arranged on at least two opposing side walls (6) of the lower part (3) of the housing (1), and each such surface has a connection zone (9, 9') extending out of the housing (1).

4. The gas sensor according to any one of claims 1 to 3, **characterised in that** the assembly opening (10) is designed in such manner that a tool arm (12) holding the sensor element (11) can be moved into an assembly position in the region of the opening perimeter (7) of the lower part (3) in such manner that the connecting wires (13, 13') of the sensor element (11) are in bondable contact with the associated solder lands (8, 8').

5. The gas sensor according to any one of the preceding claims, **characterised in that** the upper part (4) has a cover part (14) and side wall parts (15, 16), from which an outer housing with a lower opening perimeter (17) is formed.

6. The gas sensor according to any one of claims 1 to 4, **characterised in that** the lower part (3) is designed as an inner housing with an upper opening perimeter (7) .

7. The gas sensor according to claim 6, **characterised in that** an air gap is formed between the lower part (3) and the upper part (4) attached on top thereof.

8. The gas sensor according to any one of the preceding claims, **characterised in that** the cover part (14) of the upper part (4) has an opening (18) in which a gas-permeable but liquid-impermeable closure element is arranged.

9. The gas sensor according to claim 8, **characterised in that** the closure element is a membrane (19).

10. A method for producing a gas sensor, in particular for automobile applications, comprising a housing with a measuring chamber, wherein at least one sensor element having connecting wires is contained in the measuring chamber, wherein the housing has a lower part (3), and an upper part (4) which serves as a cover for the lower part (3), and each sensor element is arranged so as to be suspended by its connecting wires (13, 13') in the lower part (3),
**characterised in**
**that** an assembly opening (10) is provided in at least one of the side walls (6) of the lower part (3), and a tool arm holding the sensor element (11) is advanced from the outside into the lower part (3) through the assembly opening and moved into an assembly position in the region of the opening perimeter (7) of the lower part, so that that the connecting wires (13, 13') of the sensor element (11) are in bondable contact with the associated solder lands (8, 8'),
**that** the connecting wires (13, 13') are bonded to connection surface of the side walls (6, 7) of the lower part (3)
the tool arm (12) is withdrawn to the outside through the assembly opening (10),
the upper part (4) is arranged on the lower part (3) so that the side wall (15, 16) of the upper part (4) closes off the assembly opening (10) in the side wall (6) of the lower part (3).

## Revendications

1. Capteur de gaz, notamment pour des applications automobiles, avec un boîtier qui comporte un compartiment de mesure, dans le compartiment de mesure étant contenu au moins un élément capteur comportant des fils de raccordement, le boîtier comportant une partie inférieure (3) et une partie supérieure (4) recouvrant la partie inférieure (3) et chaque élément capteur étant placé dans la partie inférieure (3), en étant accroché à ses fils de raccordement (13, 13'),
**caractérisé en ce**
**que** dans au moins l'une de ses parois latérales (6), la partie inférieure (3) comporte une ouverture de montage qui est conçue de telle sorte qu'un bras d'outil qui maintient l'élément capteur puisse être introduit par l'extérieur, à travers l'ouverture de montage dans la partie inférieure (3) et en ce que la partie supérieure (4) est placée sur la partie inférieure (3) de telle sorte que la paroi latérale (15, 16) de la partie supérieure (4) ferme l'ouverture de montage (10) dans la paroi latérale (6) de la partie inférieure (3).

2. Capteur de gaz selon la revendication 1, **caractérisé en ce que** la partie inférieure (3) comporte un fond (5) et des parois latérales (6) partant de celui-ci dont les extrémités libres correspondant au bord d'ouverture (7) supérieur de la partie inférieure (3) du boîtier (1) .

3. Capteur de gaz selon la revendication 2, **caractérisé en ce que** sur au moins deux parois latérales (6) opposées de la partie inférieure (3) du boîtier (1) est placée au moins une surface de raccordement (8, 8') qui comportent chacune une zone de raccordement (9, 9') conduite hors du boîtier (1).

4. Capteur de gaz selon l'une quelconque des revendications 1 à 3, **caractérisé en ce que** l'ouverture de montage (10) est conçue de telle sorte qu'un bras d'outil (12) qui maintient l'élément capteur (11) puisse être amené dans une position de montage, dans la zone du bord d'ouverture (7) de la partie inférieure (3), de telle sorte que les fils de raccordement (13, 13') de l'élément capteur (11) soient en contact métallisable avec les surfaces de raccordement (8, 8') par brasage associées.

5. Capteur de gaz selon l'une quelconque des revendications précédentes, **caractérisé en ce que** la partie supérieure (4) comporte une partie formant couvercle (14) et des parties formant parois latérales (15, 16) à partir desquelles est formé un boîtier extérieur avec un bord d'ouverture (17) inférieur.

6. Capteur de gaz selon l'une quelconque des revendications 1 à 4, **caractérisé en ce que** la partie inférieure (3) est conçue sous la forme d'un boîtier intérieur avec un bord d'ouverture (7) supérieur.

7. Capteur de gaz selon la revendication 6, **caractérisé en ce qu'**entre la partie inférieure (3) et la partie supérieure (4) jointe au-dessus de celle-ci est formée une fente d'aération.

8. Capteur de gaz selon l'une quelconque des revendications précédentes, **caractérisé en ce que** la partie formant couvercle (14) de la partie supérieure (4) comporte une ouverture (18) dans laquelle est placé un élément de fermeture perméable aux gaz, mais imperméable aux liquides.

9. Capteur de gaz selon la revendication 8, **caractérisé en ce que** l'élément de fermeture est une membrane (19).

10. Procédé destiné à fabriquer un capteur de gaz, notamment pour des applications automobiles, avec un boîtier qui comporte un compartiment de mesure, dans le compartiment de mesure étant contenu au moins un élément capteur comportant des fils de raccordement, le boîtier comportant une partie inférieure (3) et une partie supérieure (4) recouvrant la partie inférieure (3) et chaque élément capteur étant placé dans la partie inférieure (3), en étant accroché à ses fils de raccordement (13, 13'),
**caractérisé en ce**
**que** dans la partie inférieure (3), dans au moins l'une de ses parois latérales (6), il est prévu une ouverture de montage (10) et on introduit par l'extérieur un bras d'outil qui maintient l'élément capteur (11), à travers l'ouverture de montage dans la partie inférieure (3) et on l'amène dans une position de montage, dans la zone du bord de montage (7) de la partie inférieure, de telle sorte que les fils de raccordement (13, 13') de l'élément capteur (11) soient en contact métallisable avec les surfaces de raccordement (8, 8') par brasage associées,
en ce qu'on métallise les fils de raccordement (13, 13') des surfaces de raccordement des parois latérales (6, 7) de la partie inférieure (3),
on retire vers l'extérieur le bras d'outil (12) à travers l'ouverture de montage (10) et
en ce qu'on place la partie supérieure (4) sur la partie inférieure (3) du boîtier, de sorte que la paroi latérale (15, 16) de la partie supérieure (4) ferme l'ouverture de montage (10) dans la paroi latérale (6) de la partie inférieure (3).
